# FASCICULE DE BREVET EUROPEEN

(11) **EP 0 704 200 B1**
(45) Date de publication et mention de la délivrance du brevet: **23.07.1997**
(21) Numéro de dépôt: 95401919.6
(22) Date de dépôt: 21.08.1995
(51) Int. Cl.: A61K 7/06, A61K 7/48

(54) **Compositions cosmétiques contenant un composé lipidique de type céramide et un peptide à une chaîne grasse, et leurs utilisations**
Kosmetische Zusammensetzungen, die eine Lipidverbindung vom Typ Ceramid und ein Peptid mit Fettkette enthalten, und deren Verwendungen
Cosmetic compositions containing a lipid ceramide-type compound and a peptide with a fatty chain

(30) Priorité: 29.09.1994 FR 9411666
(43) Date de publication de la demande: 03.04.1996
(73) Titulaire: L'OREAL, 75008 Paris (FR)
(72) Inventeur: Cauwet-Martin, Danièle, F-75011 Paris (FR); Dubief, Claude, F-78150 Le Chesnay (FR)
(74) Mandataire: Tezier Herman, Béatrice

(56) Documents cités:
- EP-A- 0 278 505
- WO-A-91/01719
- WO-A-92/21321
- WO-A-93/02656
- DE-A- 4 244 415
- DE-A- 4 326 958
- US-A- 4 751 219
- DATABASE WPI Week 9306 Derwent Publications Ltd., London, GB; AN 93-049518 & JP-A-05 000 923 (NONOGAWA SHOJI YG)

## Description

La présente invention concerne de nouvelles compositions cosmétiques comprenant dans un milieu aqueux au moins un composé lipidique de type céramide et au moins un peptide comportant au moins une chaîne grasse .

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

En effet, sous l'action de ces agressions (agents atmosphériques, traitements mécaniques ou chimiques), les cheveux perdent une partie de leurs constituants, tels que notamment des céramides et des protéines.

Les céramides ou leurs analogues sont connus pour protéger et/ou réparer la peau et/ou les fibres capillaires des agressions des divers agents et traitements cités ci-dessus. En particulier, ils ont un effet barrière qui imitent la fuite des protéines, ils renforcent également la cohésion cuticulaire (voir le document EP-A-0 278 505).

Compte tenu du fait que la protection et/ou le soin apporté par les céramides est d'autant plus élevé que leurs quantités présentes sur les cheveux ou sur la peau sont importantes, la demanderesse a donc cherché à améliorer la fixation des céramides sur et/ou dans le cheveu ou sur et/ou dans la peau.

Les céramides ou analogues présentent un caractère insoluble dans les milieux aqueux, il est donc nécessaire de les mettre en oeuvre sous une forme dispersée stable.

Ainsi, on sait émulsionner les céramides en présence de tensioactifs, en particulier certains tensioactifs cationiques. Cependant, même si ces émulsions s'avèrent stables, elles conduisent à des compositions dont les propriétés cosmétiques peuvent apparaître comme encore insuffisantes (voir le document WO-A-9 302 656).

L'invention a donc pour but d'améliorer la fixation des céramides sur et/ou dans le cheveu ou sur et/ou dans la peau.

La présente invention a également pour but de proposer des dispersions aqueuses stables à base de composés lipidiques de type céramide .

L'invention a enfin pour but de proposer des compositions cosmétiques présentant des propriétés cosmétiques amèliorées, en particulier au niveau de la tenue de la coiffure.

Or, la demanderesse a maintenant découvert qu'un peptide à au moins une chaîne grasse permettait d'atteindre ces buts.

L'invention a ainsi pour objet une composition cosmétique, caractérisée en ce qu'elle comprend, dans un milieu aqueux cosmétiquement acceptable, au moins un composé lipidique de type céramide et au moins un peptide à au moins une chaîne grasse.

L'invention a également pour objet une dispersion aqueuse, caractérisée en ce qu'elle comprend, dans un milieu aqueux, au moins un composé lipidique de type céramide et au moins un peptide à au moins une chaîne grasse.

L'invention a encore pour objet l'utilisation de cette dispersion aqueuse pour préparer la composition aqueuse selon l'invention.

L'invention concerne également l'utilisation d'un peptide à au moins une chaîne grasse comme agent de mise en dispersion d'un composé lipidique de type céramide dans une dispersion aqueuse contenant un tel composé.

Un autre objet de l'invention concerne un procédé de traitement de la peau ou des fibres kératiniques, telles que les cheveux, caractérisé en ce qu'il consiste à appliquer sur ladite peau ou sur lesdites fibres des compositions cosmétiques selon l'invention.

Les différents objets de l'invention vont maintenant être détaillés. L'ensemble des significations et définitions des composés utilisés dans la présente invention données ci-dessous sont valables pour l'ensemble des objets de l'invention.

Par composé lipidique de type céramide, on entend tout composé lipidique présentant des propriétés similaires à celles des céramides, en particulier un effet barrière et un effet renforçateur de la cohésion cuticulaire.

Ces composés peuvent posséder une structure analogue aux céramides non glycosylés naturels ou synthétiques.

Les composés lipidiques de type céramide préférés selon l'invention répondent à la formule générale (I): 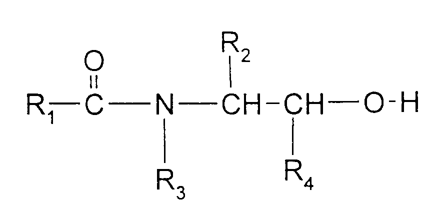 dans laquelle :
- R₁ désigne soit un radical hydrocarboné, linéaire ou ramifié, saturé ou insaturé, C₉-C₃₀, ce radical pouvant être substitué par un ou plusieurs groupements hydroxyle éventuellement estérifié par un acide gras saturé ou insaturé en C₁₆-C₃₀ ; soit un radical R''-NR-CO-R'- , R désigne l'hydrogène ou un radical hydrocarboné en C₁-C₁₀ mono ou polyhydroxylé, préférentiellement monohydroxylé, R' et R'' sont des radicaux hydrocarbonés dont la somme des atomes de carbone est comprise entre 9 et 30, R' étant un radical divalent,
- R₂ désigne un atome d'hydrogène ou un radical hydrocarboné en C₁₆-C₂₇, saturé ou insaturé, ce radical pouvant être substitué par un ou plusieurs radicaux alkyle en C₁-C₁₄; R₂ peut également désigner un radical α-hydroxyalkyle en C₁₅-C₂₆, le groupement hydroxyle étant éventuellement estérifié par un α-hydroxyacide en C₁₆-C₃₀,
- R₃ désigne un atome d'hydrogène, un radical hydrocarboné en C₁₆-C₂₇, saturé ou insaturé ou un radical -CH₂-CHOH-CH₂-O-R₆ dans lequel R₆ désigne un radical hydrocarboné en C₁₀-C_{26 ,}
- R₄ désigne un atome d'hydrogène ou un radical hydrocarboné en C₁ -C₄ mono ou polyhydroxylé.

Les composés de formule (I) préférés sont les céramides décrites par DOWNING (céramides de types 1 à 6) dans Arch. Dermatol, Vol. 123, 1381-1384, 1987, ou celles décrites dans la demande de brevet français FR-2 673 179.

Les céramides de types 1 à 6 ont la structure suivante :

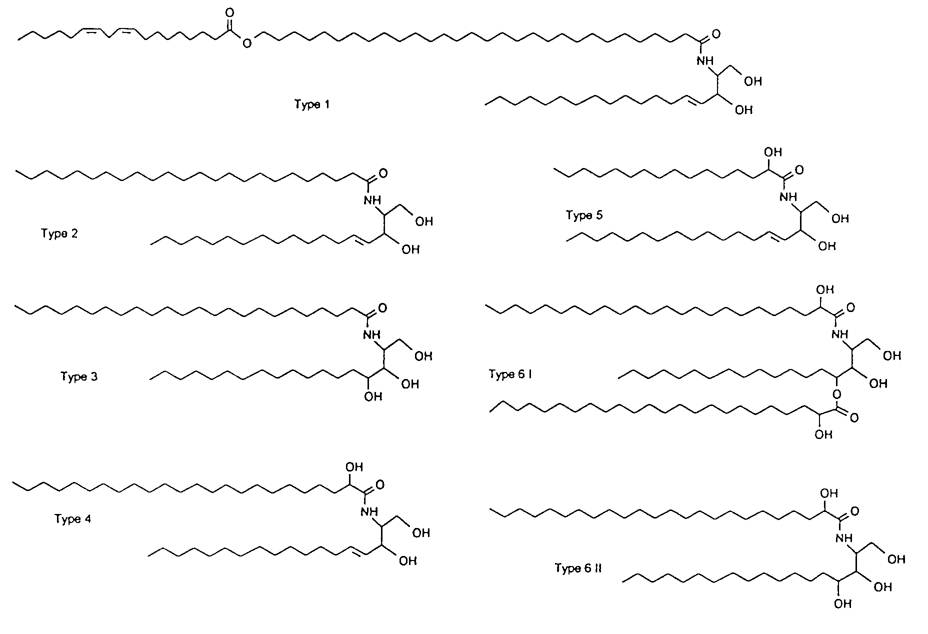

Les céramides plus particulièrement préférés selon l'invention sont les composés de formule (I) pour lesquels R₁ désigne un alkyle saturé ou insaturé dérivé d'acides gras en C₁₆-C₂₂ et R₂ désigne un radical linéaire hydrocarboné saturé en C₁₅.

De tels composés sont par exemple :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

On peut également utiliser les composés de formule (I) décrits dans les demandes de brevet EP-A-0227994 et WO94/07844. De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique .

On peut également utiliser le N-docasanoyl N-méthyl-D-glucamine décrit dans la demande de brevet WO92/05764.

La concentration en composé lipidique de type céramide dans la composition cosmétique selon l'invention varie préférentiellement entre 0,0001% et 10% en poids par rapport au poids total de la composition, et de préférence entre 0,0001 et 5%.

La présente invention a également pour objet une dispersion aqueuse, caractérisée en ce qu'elle comprend au moins un composé lipidique de type céramide et au moins un peptide à au moins une chaîne grasse.

Cette dispersion aqueuse présente l'avantage d'être stable et peut donc facilement être utilisée pour préparer la composition cosmétique selon la présente invention.

La dispersion aqueuse selon l'invention présente préférentiellement une concentration en composé lipidique de type céramide variant entre 0,0001% et 15% en poids par rapport au poids total de ladite dispersion, et de préférence entre 0,0001 et 10%.

Par peptide, on entend tout composé présentant au moins une liaison peptidique, tels que notamment les protéines ou les hydrolysats de protéines.

Les protéines à au moins une chaîne grasse utilisables dans la présente invention sont soit d'origine naturelle, soit d'origine synthétique. Les hydrolysats de protéine à au moins une chaîne grasse correspondent à des protéines hydrolysées, la ou les chaînes grasses venant des protéines hydrolysées ou étant apportées après hydrolyse.

La ou les chaînes grasses des peptides de l'invention sont introduites, si elles ne sont pas présentes naturellement, par modification chimique telle que par greffage ou par quaternisation.

La ou les chaînes grasses des peptides selon l'invention peuvent comprendre de 8 à 40 atomes de carbone et de préférence de 10 à 22 atomes de carbone. On peut par exemple citer les chaînes cocoyle, lauryle ou stéaryle.

Les peptides à au moins une chaîne grasse selon l'invention ont avantageusement un poids moléculaire supérieur à 1000.

Les protéines utilisables selon l'invention peuvent être issues de matières d'origine animale ou végétale.

A titre de protéines d'origine animale, on peut notamment citer la kératine, l'élastine, le collagène, les protéines extraites du lait telles que la lactoferrine, la caséine, le caséinate de sodium, de magnésium ou de calcium, les protéines du babeurre, les protéines du lactosérum dont l'α-lactalbumine, la β-lactoglobuline et les immunoglobulines, l'albumine de blanc d'oeuf.

A titre d'exemples de protéines d'origine végétale, on peut notamment citer les protéines extraites du blé, de germes de blé, de l'avoine, de l'orge, du maïs, du riz, du soja, de fèves, de graines de coton, de graines de lupin, de pommes de terre, de noyaux d'abricots.

Dans ces exemples, certaines protéines ne présentent pas de chaîne grasse, il faut alors les modifier chimiquement pour qu'elles en présentent.

On préfère utiliser, dans la présente invention, les hydrolysats de protéine à au moins une chaîne grasse.

Ainsi, à titre d'hydrolysats de protéine à au moins une chaîne grasse convenant particulièrement bien pour la présente invention, on peut notamment citer :
- les hydrolysats de kératine de laine portant des groupements N-hydroxypropyl diméthyl cocoylamidopropyl ammonium tels que ceux vendus sous la dénomination "MONTEINE LKA"® par la société SEPPIC, ou des groupements cocoyl diméthyl N-hydroxypropyl ammonium tels que ceux vendus sous les dénominations "CROQUAT WKP"® par la société CRODA, ou des groupements stéaryl diméthyl N-hydroxypropyl ammonium tels que ceux vendus sous la dénomination "PROMOIS WK-HSAQ"® par la société SEIWA KASEI,
- les hydrolysats de kératine portant des groupements lauryl diméthyl ammonium tels que ceux vendus sous la dénomination "CROQUAT K" par la société CRODA,
- les hydrolysats de caséine portant des groupements des groupements lauryl diméthyl ammonium tels que ceux vendus sous la dénomination "HYDROLACTIN QL"® par la société CRODA,
- les hydrolysats de fibroïne de soie portant des groupements cocoyl diméthyl N-hydroxypropyl ammonium tels que ceux vendus sous les dénominations "PROMOIS SILK-CAQ"® par la société SEIWA KASEI ou "CROSILKQUAT"® par la société CRODA, ou des groupements lauryl diméthyl N-hydroxypropyl ammonium tels que ceux vendus sous la dénomination "PROMOIS SILK-LAQ"® par la société SEIWA KASEI, ou des groupements stéaryl diméthyl N-hydroxypropyl ammonium tels que ceux vendus sous la dénomination "PROMOIS SlLK-SAQ"® par la société SEIWA KASEI,
- les hydrolysats de collagène portant des groupements N-hydroxypropyl diméthyl laurylamidopropyl ammonium tels que ceux vendus sous la dénomination "MONTEINE LCQ"® par la société SEPPIC, ou des groupements N-hydroxypropyl cocoyl diméthyl ammonium tels que ceux vendus sous la dénomination "LEXEIN QX 3000"® par la société INOLEX, ou des groupements stéaryl triméthyl ammonium tels que ceux vendus sous la dénomination "QUAT-COLL QS"® par la société QUIMDIS,
- les hydrolysats de protéines de blé portant des groupements stéaryl diméthyl N-hydroxypropyl ammonium tels que ceux vendus sous la dénomination "HYDROTITICUM QS"® par la société CRODA, ou des groupements lauryl diméthyl N-hydroxypropyl ammonium tels que ceux vendus sous la dénomination "HYDROTITICUM QL"® par la société CRODA, ou des groupements cocoyl diméthyl N-hydroxypropyl ammonium tels que ceux vendus sous la dénomination "HYDROTITICUM QM"® par la société CRODA,
- les hydrolysats de protéines de soja portant des groupements lauryl diméthyl N-hydroxypropyl ammonium tels que ceux vendus sous les dénominations "PROMOIS WS-LAQ"® par la société SEIWA KASEI ou "CROQUAT SOYA"® par la société CRODA, ou des groupements cocoyl diméthyl ammonium tels que ceux vendus sous la dénomination "PROMOIS WS-CAQ"® par la société SEIWA KASEI.

La concentration en peptide à au moins une chaîne grasse dans la composition cosmétique selon l'invention varie préférentiellement entre 0,01% et 30% en poids par rapport au poids total de la composition, et de préférence entre 0,1 et 10% en poids.

La dispersion aqueuse selon l'invention, qui peut être notamment utilisée pour préparer la composition cosmétique selon l'invention, présente préférentiellment une concentration en peptides à au moins une chaîne grasse variant entre 0,01% et 40% en poids par rapport au poids total de ladite dispersion, et de préférence entre 0,5 et 20%.

Le rapport des concentrations en poids peptide/ composé lipidique de type céramide dans la composition cosmétique, comme dans la dispersion aqueuse, est avantageusement compris entre 1,5 et 10 et de préférence entre 2 et 6.

La composition de l'invention peut également contenir au moins un additif choisi parmi les épaississants, les silicones volatiles ou non volatiles, solubles ou insolubles, les tensioactifs, les parfums, les agents nacrants, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est déterminée facilement par l'homme de l'art selon sa nature et sa fonction.

L'invention a encore pour objet un procédé de traitement de la peau ou des fibres kératiniques, telles que les cheveux, caractérisé en ce qu'il consiste à appliquer sur la peau ou sur les fibres kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage de la peau, des cheveux ou de toute autre matière kératinique.

Les compositions cosmétiques selon l'invention peuvent se présenter sous forme de gel, de lait, de crème, de lotion plus ou moins épaissie ou de mousse et être utilisées pour la peau ou les cheveux.

Pour les cheveux, elles sont plus particulièrement des shampooings, des compositions à rincer ou non, à appliquer avant ou après un shampooing, une coloration, une décoloration, une permanente ou un défrisage, des compositions de coloration, de décoloration, de permanente ou de défrisage des cheveux.

Les compositions peuvent être également des lotions de mise en plis, des lotions pour le brushing, des compositions de fixation (laques) et de coiffage. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Dans tout ce qui suit ou ce qui précède, les pourcentages exprimés sont en poids.

L'invention va être maintenant plus complètement illustrée à l'aide des exemples suivants.

Dans les exemples, MA signifie matière active.

### EXEMPLE 1

On a préparé une composition 1A ayant la formulation suivante :

| | |
|---|---|
| - N-oléoyl dihydrosphingosine | 0,1 g |
| - Hydrolysat de kératine de laine quaternisé par du chlorure de N-cocoyl diméthyl ammonium en solution aqueuse à 30% MA (vendu sous la dénomination CROQUAT WKP® par la société CRODA) | 0,5 gMA |
| - Eau qsp | 100 g |
| Le pH est ajusté à 5. | |

On a préparé une composition 1B (comparatif) ayant la formulation suivante :

| | |
|---|---|
| - N-oléoyl dihydrosphingosine | 0,1 g |
| - Quaternium-27 (REWOQUAT W75 PG® de REWO) | 0,5 gMA |
| - Eau qsp | 100 g |
| Le pH est ajusté à 5. | |

Le mode opératoire est le suivant : on applique 5 g de la composition ci-dessus sur une mèche de cheveux de 2,5 g; puis on laisse poser pendant 10 minutes, ensuite la mèche est rincée 3 fois en effectuant à chaque fois 2 passages dans 200 ml d'eau. La mèche est ensuite séchée avec un sèche-cheveux.

Pour comparer la quantité de composé lipidique de type céramide fixée sur les cheveux, on applique la même quantité de composition 1B ci-dessus sur une mèche de cheveux de même qualité . Le mode opératoire est identique.

Chaque mèche est divisée en deux et on extrait le céramide par extraction au dichlorométhane (2 fois 20 ml pendant 1 heure à température ambiante sous agitation mécanique). Les amides sont ensuite séparés des lipides du cheveu par CCM (Chromatographie sur Couche Mince). Le céramide est ensuite dosé par photodensitométrie après carbonisation avec une solution à 3% en poids d'acide sulfurique puis passage à l'étuve à 180°C (on utilise une quantité connue de céramide comme référence).

Les résultats sont rassemblés dans le tableau suivant:

| | Quantité de céramide extraite par g de cheveux |
|---|---|
| Composition 1A (selon l'invention) | 229 µg |
| Composition 1B (comparatif) | 149 µg |

La quantité de céramide fixée sur les cheveux est beaucoup plus importante avec la composition selon l'invention.

On a également comparé la nervosité de deux mèches de cheveux de 2,5 g traitées respectivement avec la composition 1A ou 1B.
La nervosité est évaluée en fonction du replacement des cheveux ondulés dans leur position initiale après le passage d'un peigne ou d'une brosse.
On a appliqué 5 g de composition sur une mèche de cheveux de 2,5 g; puis on laisse poser pendant 10 minutes, ensuite la mèche est rincée 3 fois en effectuant à chaque fois 2 passages dans 200 ml d'eau. La mèche est ensuite enroulés sur des bigoudis puis séchée avec un sèche-cheveux.
On a ensuite demandé à un panel de dix testeurs quelle était la mèche la plus nerveuse. Les dix testeurs ont désigné la mèche traitée avec la composition 1A selon l'invention.

### EXEMPLE 2

On a préparé une mousse de soin capillaire de composition suivante :

| | |
|---|---|
| Hydrolysat de protéine de blé portant une chaîne grasse isostéaryle en solution alcoolique à 34% de matière active (MA) vendu sous la dénomination CROTEIN ADW® par la société CRODA | 0,3 gMA |
| Céramide de type 5 | 0,1 g |
| Conservateur, parfum qs | |
| HCl qs pH 8 | |
| Eau qsp | 100 g |

### Shéma de pressurisation :

| | |
|---|---|
| Composition ci-dessus : | 90 g |
| Mélange ternaire de n-butane, isobutane et propane (23/55/22), vendu sous la dénomination "AEROGAZ 3,2 N par la société ELF AQUITAINE | 10 g |

Appliquée sur cheveux mouillés, cette mousse fond rapidement dans les cheveux et améliore le démêlage des cheveux mouillés. Les cheveux séchés obtenus sont nerveux et doux.

### EXEMPLE 3

On a préparé un après-shampooing à rincer de composition suivante :

| | |
|---|---|
| Hydrolysat de kératine de laine quaternisé par du chlorure de N-cocoyl diméthyl ammonium en solution aqueuse à 30% de MA vendu sous la dénomination CROQUAT WKP® par la société CRODA | 0,5 gMA |
| N-oléoyldihydrosphingosine | 0,1 g |
| Copolymère de vinylpyrrolidone / méthacrylate de diméthyl amino éthyle en solution aqueuse à 20% vendu sous la dénomination Copolymère 845® par la société ISP | 1 gMA |
| Copolymère acrylamide / chlorure de méthacrylate d'éthyle triméthyl ammonium (52/48) réticulé en dispersion à 50% dans une huile minérale (SALCARE SC 92® de ALLIED COLLOID) | 2 gMA |
| Conservateur, parfum qs | |
| NaOH qs pH 4,5 | |
| Eau qsp | 100 g |

Appliquée sur cheveux mouillés et rincés, après un temps de pause, ce soin améliore le démêlage des cheveux mouillés et apporte lissage et douceur sur cheveux séchés.

### EXEMPLE 4

On a préparé une lotion de composition suivante :

| | |
|---|---|
| N-(2-hydroxyéthyl)-N-(3-cétyloxy-2-hydroxypropyl)amide d'acide cétylique | 0,1 g |
| Hydrolysat de kératine de laine quaternisé par du chlorure de N-cocoyl diméthyl ammonium en solution aqueuse à 30 % de MA vendu sous la dénomination CROQUAT WKP® par la société CRODA | 0,5 gMA |
| Conservateur, parfum qs | |
| Eau qsq | 100 g |
| pH spontané 4,2 | |

Cette lotion est appliquée sur cheveux mouillés. Après rinçage, les cheveux sont faciles à démêler et souples.

### EXEMPLE 5

On a préparé une lotion de composition suivante :

| | |
|---|---|
| Bis (N-hydroxyéthyl N-cétyl) malonamide vendu par Quest International sous le nom de Questamide H® | 0,1 g |
| Hydrolysat de kératine de laine quaternisé par du chlorure de N-cocoyl diméthyl ammonium en solution aqueuse à 30 % de MA vendu sous la dénomination CROQUAT WKP® par la société CRODA | 0,5 gMA |
| Conservateur, parfum qs | |
| Eau qsq | 100 g |
| pH spontané 4,5 | |

Cette lotion, appliquée sur cheveux lavés, apporte de la douceur sur cheveux mouillés et un toucher gainé lisse sur cheveux séchés.

### EXEMPLE 6

| | |
|---|---|
| Céramide de type 2 | 10 g |
| Hydrolysat de kératine de laine quaternisé par du chlorure de N-cocoyl diméthyl ammonium en solution aqueuse à 30 % de MA vendu sous la dénomination CROQUAT WKP® par la société CRODA | 24 gMA |
| Conservateur, parfum qs | |
| Eau qsq | 100 g |
| pH spontané 4 | |

Cette crème épaisse s'applique sur les cheveux mouillés et leur confère, après rinçage, de bonnes propriétés cosmétiques (démêlage et douceur).

## Revendications

1. Composition cosmétique, caractérisée par le fait qu'elle comprend, dans un milieu aqueux cosmétiquement acceptable, au moins un composé lipidique de type céramide et au moins un peptide à au moins une chaîne grasse.

2. Composition selon la revendication 1, caractérisée en ce que le composé lipidique de type céramide présente une formule générale (I): 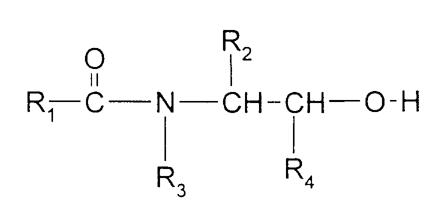 dans laquelle :
- R₁ désigne soit un radical hydrocarboné, linéaire ou ramifié, saturé ou insaturé, C₉-C₃₀, ce radical pouvant être substitué par un ou plusieurs groupements hydroxyle éventuellement estérifié par un acide gras saturé ou insaturé en C₁₆-C₃₀ ; soit un radical R''-NR-CO-R'- , R désigne l'hydrogène ou un radical hydrocarboné en C₁-C₁₀ mono ou polyhydroxylé, R' et R'' sont des radicaux hydrocarbonés dont la somme des atomes de carbone est comprise entre 9 et 30, R' étant un radical divalent.
- R₂ désigne un atome d'hydrogène ou un radical hydrocarboné en C₁₆-C₂₇, saturé ou insaturé, ce radical pouvant être substitué par un ou plusieurs radicaux alkyle en C₁-C₁₄; R₂ peut également désigner un radical α-hydroxyalkyle en C₁₅-C₂₆, le groupement hydroxyle étant éventuellement estérifié par un α-hydroxyacide en C₁₆-C₃₀.
- R₃ désigne un atome d'hydrogène, un radical hydrocarboné en C₁₆-C₂₇, saturé ou insaturé ou un radical -CH₂-CHOH-CH₂-O-R₆ dans lequel R₆ désigne un radical hydrocarboné en C₁₀-C₂₆
- R₄ désigne un atome d'hydrogène ou un radical hydrocarboné en C₁-C₄ mono ou polyhydroxylé.

3. Composition selon la revendication 2, caractérisée en ce que le composé répond à la formule (I) dans laquelle R₁ désigne un radical saturé ou insaturé dérivé d'acide gras en C₁₆-C₂₂ et R₂ désigne un radical linéaire hydrocarboné saturé en C₁₅.

4. Composition selon la revendication précédente, caractérisé en ce que le composé de formule (I) est choisi parmi la N-linoléoyldihydrosphingosine, la N-oléoyldihydrosphingosine, la N-palmitoyldihydrosphingosine, la N-stéaroyldihydrosphingosine, la N-béhénoyldihydrosphingosine ou les mélanges de ces composés.

5. Composition selon la revendication 2, caractérisée en ce que le composé de formule (I) est choisi parmi le bis-(N-hydroxyéthyl N-cétyl) malonamide, le N-(2-hydroxyéthyl)-N-(3-cétyloxy-2-hydroxypropyl)amide d'acide cétylique) et le N-docasanoyl N-méthyl-D-glucamine.

6. Composition selon l'une quelconque des revendications précédentes, caractérisée par le fait que le composé lipidique de type céramide est présent dans des concentrations allant de 0,0001 à 10% en poids par rapport au poids total de la composition, de préférence de 0,0001 à 5%.

7. Composition selon l'une quelconque des revendications précédentes, caractérisée par le fait que la ou les chaînes grasses des peptides comprennent de 8 à 40 atomes de carbone et de préférence de 10 à 22 atomes de carbone

8. Composition selon l'une quelconque des revendications précédentes, caractérisée par le fait que les peptides à chaîne grasse ont un poids moléculaire supérieur à 1000.

9. Composition selon l'une quelconque des revendications précédentes, caractérisée en ce que les peptides sont des protéines ou des hydrolysats de protéines à chaîne grasse.

10. Composition selon l'une quelconque des revendications précédentes, caractérisée en ce que l'on utilise des hydrolysats de protéines à chaîne grasse.

11. Composition selon l'une quelconque des revendications précédentes, caractérisée par le fait que la concentration en peptide est comprise entre 0,01 et 30%, de préférence entre 0,1 et 10%, en poids par rapport à l'ensemble de la composition.

12. Composition selon l'une des revendications précédentes, caractérisée en ce que le rapport des concentrations en poids peptide / composé lipidique de type céramide est compris entre 1,5 et 10 et de préférence entre 2 et 6.

13. Dispersion aqueuse, caractérisée en ce qu'elle comprend au moins un composé lipidique de type céramide et au moins un peptide à au moins une chaîne grasse.

14. Dispersion aqueuse selon la revendication précédente, caractérisée en ce que le composé lipidique de type céramide est celui défini selon l'une des revendications 2 à 5.

15. Dispersion selon l'une des revendications 13 ou 14, caractérisée en ce que la concentration en composé lipidique de type céramide varie entre 0,0001 et 15%, de préférence entre 0,0001 et 10%, en poids par rapport au poids total de ladite dispersion.

16. Dispersion selon l'une des revendications 13 à 15, caractérisée en ce que le peptide à au moins une chaîne grasse est tel que défini dans les revendications 7 à 10.

17. Dispersion selon l'une des revendications 13 à 15, caractérisée par le fait que la concentration en peptides est comprise entre 0,01 et 40%, de préférence entre 0,5 et 20%, en poids par rapport au poids total de ladite dispersion.

18. Dispersion selon l'une des revendications 13 à 17, caractérisée en ce que le rapport des concentrations en poids peptide / composé lipldique de type céramide est compris entre 1,5 et 10 et de préférence entre 2 et 6.

19. Utilisation de la dispersion aqueuse selon l'une des revendications 13 à 18 pour préparer la composition cosmétique selon l'une des revendications 1 à 12.

20. Utilisation d'un peptide à au moins une chaîne grasse comme agent de mise en dispersion d'un composé lipidique de type céramide dans une dispersion aqueuse contenant un tel composé.

21. Procédé de traitement de la peau ou des fibres kératiniques, telles que les cheveux, caractérisé en ce qu'il consiste à appliquer sur ladite peau ou sur lesdites fibres des compositions cosmétiques selon l'une des revendications 1 à 12.

## Claims

1. Cosmetic composition, characterized in that it comprises, in a cosmetically acceptable aqueous medium, at least one lipid compound of the ceramide type and at least one peptide having at least one fatty chain.

2. Composition according to Claim 1, characterized in that the lipid compound of the ceramide type has a general formula (I): 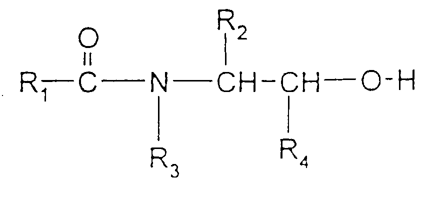 in which:
- R₁ denotes either a saturated or unsaturated, linear or branched C₉-C₃₀ hydrocarbon radical, it being possible for this radical to be substituted with one or more hydroxyl groups optionally esterified with a saturated or unsaturated C₁₆-C₃₀ fatty acid; or a radical R''-NR-CO-R'-, R denotes hydrogen or a mono- or polyhydroxylated C₁-C₁₀ hydrocarbon radical, R' and R'' are hydrocarbon radicals in which the sum of the carbon atoms is between 9 and 30, R' being a bivalent radical,
- R₂ denotes a hydrogen atom or a saturated or unsaturated C₁₆-C₂₇ hydrocarbon radical, it being possible for this radical to be substituted with one or more C₁-C₁₄ alkyl radicals; R₂ can also denote a C₁₅-C₂₆ α-hydroxyalkyl radical, the hydroxyl group being optionally esterified with a C₁₆-C₃₀ α-hydroxy acid,
- R₃ denotes a hydrogen atom, a saturated or unsaturated C₁₆-C₂₇ hydrocarbon radical or a radical -CH₂-CHOH-CH₂-O-R₆ in which R₆ denotes a C₁₀-C₂₆ hydrocarbon radical,
- R₄ denotes a hydrogen atom or a mono- or polyhydroxylated C₁-C₄ hydrocarbon radical.

3. Composition according to Claim 2, characterized in that the compound corresponds to the formula (I) in which R₁ denotes a saturated or unsaturated radical derived from a C₁₆-C₂₂ fatty acid, and R₂ denotes a saturated linear C₁₅ hydrocarbon radical.

4. Composition according to the preceding claim, characterized in that the compound of formula (I) is chosen from N-linoleoyldihydrosphingosine, N-oleoyldihydrosphingosine, N-palmitoyldihydrosphingosine, N-stearoyldihydrosphingosine, N-behenoyldihydrosphingosine or mixtures of these compounds.

5. Composition according to Claim 2, characterized in that the compound of formula (I) is chosen from bis(N-hydroxyethyl-N-cetyl)malonamide, cetylic acid N-(2-hydroxyethyl)-N-(3-cetyloxy-2-hydroxypropyl)amide and N-docosanoyl-N-methyl-D-glucamine.

6. Composition according to any one of the preceding claims, characterized in that the lipid compound of the ceramide type is present in concentrations ranging from 0.0001 to 10% by weight relative to the total weight of the composition, and preferably from 0.0001 to 5%.

7. Composition according to any one of the preceding claims, characterized in that the fatty chain or chains of the peptides comprise from 8 to 40 carbon atoms, and preferably from 10 to 22 carbon atoms.

8. Composition according to any one of the preceding claims, characterized in that the peptides having a fatty chain have a molecular weight above 1000.

9. Composition according to any one of the preceding claims, characterized in that the peptides are proteins or protein hydrolysates having a fatty chain.

10. Composition according to any one of the preceding claims, characterized in that protein hydrolysates having a fatty chain are used.

11. Composition according to any one of the preceding claims, characterized in that the concentration of peptide is between 0.01 and 30%, and preferably between 0.1 and 10%, by weight relative to the whole composition.

12. Composition according to any one of the preceding claims, characterized in that the ratio of the concentrations by weight of peptide to lipid compound of the ceramide type is between 1.5 and 10, and preferably between 2 and 6.

13. Aqueous dispersion, characterized in that it comprises at least one lipid compound of the ceramide type and at least one peptide having at least one fatty chain.

14. Aqueous dispersion according to the preceding claim, characterized in that the lipid compound of the ceramide type is the one defined according to one of Claims 2 to 5.

15. Dispersion according to either of Claims 13 and 14, characterized in that the concentration of lipid compound of the ceramide type varies between 0.0001 and 15%, and preferably between 0.0001 and 10%, by weight relative to the total weight of the said dispersion.

16. Dispersion according to one of Claims 13 to 15, characterized in that the peptide having at least one fatty chain is as defined in Claims 7 to 10.

17. Dispersion according to one of Claims 13 to 15, characterized in that the concentration of peptides is between 0.01 and 40%, and preferably between 0.5 and 20%, by weight relative to the total weight of the said dispersion.

18. Dispersion according to one of Claims 13 to 17, characterized in that the ratio of the concentrations by weight of peptide to lipid compound of the ceramide type is between 1.5 and 10, and preferably between 2 and 6.

19. Use of the aqueous dispersion according to one of Claims 13 to 18 for preparing the cosmetic composition according to one of Claims 1 to 12.

20. Use of a peptide having at least one fatty chain as an agent for dispersing a lipid compound of the ceramide type in an aqueous dispersion containing such a compound.

21. Process for treating the skin or keratinous fibres such as hair, characterized in that it consists in applying cosmetic compositions according to one of Claims 1 to 12 to the said skin or to the said fibres.

## Patentansprüche

1. Kosmetische Zusammensetzungen,
dadurch gekennzeichnet, daß
sie in einem kosmetisch akzeptablen Medium mindestens eine Lipidverbindung vom Typ Ceramid und mindestens ein Peptid mit mindestens einer Fettkette enthalten.

2. Zusammensetzungen nach Anspruch 1, dadurch gekennzeichnet, daß die Lipidverbindung vom Typ Ceramid die allgemeine Formel (I) aufweist: 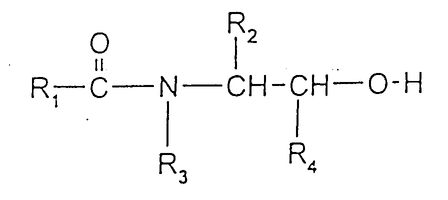 worin bedeuten:
- R₁ entweder eine geradkettige oder verzweigte gesättigte oder ungesättigte C₉₋₃₀-Kohlenwasserstoffgruppe, wobei die Gruppe mit einer oder mehreren ggf. mit einer gesättigten oder ungesättigten C₁₆₋₃₀-Fettsäure veresterten Hydroxygruppe substituiert sein kann, oder eine Gruppe R''-NR-CO-R'-, wobei R Wasserstoff oder eine C₁₋₁₀-Kohlenwasserstoffgruppe mit einer oder mehreren Hydroxygruppen und vorzugsweise einer Hydroxygruppe bedeutet und R' und R'' Kohlenwasserstoffgruppen sind, deren Summe an Kohlenstoffatomen im Bereich von 9 bis 30 liegt, wobei R' eine zweiwertige Gruppe ist,
- R₂ Wasserstoff oder eine gesättigte oder ungesättigte C₁₆₋₂₇-Kohlenwasserstoffgruppe, wobei die Gruppe mit einer oder mehreren C₁₋₁₄-Alkylgruppen substituiert sein kann; R₂ kann ferner eine C₁₅₋₂₆-α-Hydroxyalkylgruppe bedeuten, wobei die Hydroxygruppe ggf. mit einer C₁₆₋₃₀-α-Hydroxysäure verestert ist,
- R₃ Wasserstoff, eine gesättigte oder ungesättigte C₁₆₋₂₇-Kohlenwasserstoffgruppe oder eine Gruppe -CH₂-CHOH-CH₂-O-R₆, wobei R₆ eine C₁₀₋₂₆-Kohlenwasserstoffgruppe bedeutet,
- R₄ Wasserstoff oder eine C₁₋₄-Kohlenwasserstoffgruppe mit einer oder mehreren Hydroxygruppen.

3. Zusammensetzungen nach Anspruch 2, dadurch gekennzeichnet, daß die Verbindung der Formel (I) entspricht, worin R₁ eine gesättigte oder ungesättigte von einer C₁₆₋₂₂-Fettsäure abgeleitete Gruppe und R₂ eine geradkettige gesättigte C₁₅-Kohlenwasserstoffgruppe bedeutet.

4. Zusammensetzungen nach dem vorhergehenden Anspruch, dadurch gekennzeichnet, daß die Verbindung der Formel (I) unter N-Linoleoyldihydrosphingosin, N-Oleyldihydrosphingosin, N-Palmitoyldihydrosphingosin, N-Stearoyldihydrosphingosin, N-Behenoyldihydrosphingosin oder den Gemischen dieser Verbindungen ausgewählt ist.

5. Zusammensetzungen nach Anspruch 2, dadurch gekennzeichnet, daß die Verbindung der Formel (I) unter Bis(N-hydroxyethyl-N-cetyl)malonamid, N-(2-Hydroxyethyl)-N-(3-cetyloxy-2-hydroxypropyl)-Cetylsäureamid und N-Docasanoyl-N-methyl-D-glucamin ausgewählt ist.

6. Zusammensetzungen nach einem der vorhergehenden Ansprüche, dadurch gekennzeichnet, daß die Lipidverbindung vom Typ Ceramid in Konzentrationen im Bereich von 0,0001 bis 10 Gew.-%, bezogen auf das Gesamtgewicht der Zusammensetzung, und vorzugsweise im Breich von 0,0001 bis 5 Gew.-% vorliegt.

7. Zusammensetzungen nach einem der vorhergehenden Ansprüche, dadurch gekennzeichnet, daß die Fettkette oder die Fettketten der Peptide 8 bis 40 und vorzugsweise 10 bis 22 Kohlenstoffatome enthalten.

8. Zusammensetzungen nach einem der vorhergehenden Ansprüche, dadurch gekennzeichnet, daß die Peptide mit Fettkette ein Molekulargewicht über 1000 aufweisen.

9. Zusammensetzungen nach einem der vorhergehenden Ansprüche, dadurch gekennzeichnet, daß die Peptide Proteine oder Proteinhydrolysate mit Fettkette sind.

10. Zusammensetzungen nach einem der vorhergehenden Ansprüche, dadurch gekennzeichnet, daß Proteinhydrolysate mit Fettkette verwendet werden.

11. Zusammensetzungen nach einem der vorhergehenden Ansprüche, dadurch gekennzeichnet, daß die Konzentration der Peptide im Bereich von 0,01 bis 30 Gew.-% und vorzugsweise 0,1 bis 10 Gew.-%, bezogen auf die gesamte Zusammensetzung, liegt.

12. Zusammensetzungen nach einem der vorhergehenden Ansprüche, dadurch gekennzeichnet, daß das Verhältnis der gewichtsbezogenen Konzentrationen von Peptid zu Lipidverbindung vom Typ Ceramid im Bereich von 1,5 bis 10 und vorzugsweise 2 bis 6 liegt.

13. Wäßrige Dispersionen, dadurch gekennzeichnet, daß sie mindestens eine Lipidverbindung vom Typ Ceramid und mindestens ein Peptid mit mindestens einer Fettkette enthalten.

14. Wäßrige Dispersionen nach dem vorhergehenden Anspruch, dadurch gekennzeichnet, daß die Lipidverbindung vom Typ Ceramid eine Verbindung nach einem der Ansprüche 2 bis 5 ist.

15. Dispersionen nach einem der Ansprüche 13 oder 14, dadurch gekennzeichnet, daß die Konzentration der Lipidverbindung vom Typ Ceramid im Bereich von 0,0001 bis 15 Gew.-% und vorzugsweise 0,0001 bis 10 Gew.-%, bezogen auf das Gesamtgewicht der Dispersion, liegt.

16. Dispersionen nach einem der Ansprüche 13 bis 15, dadurch gekennzeichnet, daß das Peptid mit mindestens einer Fettkette ein Peptid nach den Ansprüchen 7 bis 10 ist.

17. Dispersionen nach einem der Ansprüche 13 bis 15, dadurch gekennzeichnet, daß die Konzentration der Peptide im Bereich von 0,01 bis 40 Gew.-% und vorzugsweise 0,5 bis 20 Gew.-%, bezogen auf das Gesamtgewicht der Dispersion, liegt.

18. Dispersionen nach einem der Ansprüche 13 bis 17, dadurch gekennzeichnet, daß das Verhältnis der gewichtsbezogenen Konzentrationen von Peptid zu Lipidverbindung vom Typ Ceramid im Bereich von 1,5 bis 10 und vorzugsweise im Bereich von 2 bis 6 liegt.

19. Verwendung der wäßrigen Dispersionen nach einem der Ansprüche 13 bis 18 zur Herstellung der kosmetischen Zusammensetzungen nach einem der Ansprüche 1 bis 12.

20. Verwendung eines Peptids mit mindestens einer Fettkette als Mittel zur Dispergierung einer Lipidverbindung vom Typ Ceramid in einer wäßrigen Dispersion, die diese Verbindung enthält.

21. Verfahren zur Behandlung der Haut oder von Keratinfasern, wie dem Haar, dadurch gekennzeichnet, daß es darin besteht, auf die Haut oder die Fasern eine kosmetische Zusammensetzung nach einem der Ansprüche 1 bis 12 aufzutragen.
